Europäisches Patentamt

European Patent Office

Office européen des brevets

(11) Publication number: **0 014 995**
A2

(12)

# EUROPEAN PATENT APPLICATION

(21) Application number: **80100834.3**

(22) Date of filing: **20.02.80**

(51) Int. Cl.³: **A 61 K 39/00**
//A61K39/39, A61K39/395,
G01N33/54

(30) Priority: **21.02.79 SE 7901565**

(43) Date of publication of application:
**03.09.80 Bulletin 80/18**

(84) Designated Contracting States:
**BE CH DE FR GB NL SE**

(71) Applicant: **PHARMACIA AB**
**Box 181**
**S-751 04 Uppsala(SE)**

(72) Inventor: **Uhlin, Anders Gustav**
**Graalsvägen 18**
**S-753 50 Uppsala(SE)**

(74) Representative: **Kraus, Walter, Dr. et al,**
**Patentanwälte Dres. Kraus & Weisert Irmgardstrasse 15**
**D-8000 München 71(DE)**

(54) Immunisation agent, a method of preparing the same and use thereof for producing antisera.

(57) The invention relates to an agent for immunisation which contains a particulate complex between hyaluronic acid and a proteinous antigen. The invention further relates to a method of preparing this particulate complex and to a method of producing antisera by injection of the particulate complex into a host animal.

EP 0 014 995 A2

Immunisation agent, a method of preparing the same and use thereof for
producing antisera.

The present invention relates to immunisation agents and preparations containing a particulate complex between hyaluronic acid and antigenic
proteins. The invention further relates to a method of preparing such agents
and to the use thereof for preparing antisera. In this context the term
antigenic proteins refers to all proteins capable of inducing the production of
antibodies in a host animal, such as allergens, vaccines, cell antigens, etc..

It is known that injection of suitable amounts of antigenic proteins
into humans and animals gives rise to production of the corresponding
antibodies thereby creating an immune defense against the respective antigen.
By injection of antigens it is thus possible to obtain immunity against various
allergies and deseases. When injecting only the respective antigens the immune
response is, however, comparatively weak and the effect is comparatively
short.This i.a. means that the treatment has to be repeated several times, e.g.
up to thirty times or more for allergens. Attempts have been made to
eliminate these drawbacks by combining the antigens with various adjuvants.
Typical examples of such adjuvants are Freund's adjuvant and aluminum
hydroxide. Further, conjugates of antigens with certain polysaccharides are
known (see for example British patent specification 1,174,854). Although these
adjuvants contribute to an improved immune response they have severa.
drawbacks limiting the practical utility thereof, and the purpose of the present
invention is in the first place to eliminate or reduce these drawbacks.

It has according to the invention surprisingly been found that
injection of a complex of an antigen with hyaluronic acid (which as such is a a
non-antigenic substance naturally occurring in the body) gives rise to a strong
and long lasting immune response already at very small doses of the antigen, if
the complex is used in particulate form. When injecting such a particulate
complex the antigen in question is released slowly (depot effect) and is
resorbed extremely well. Further, non-desired side effects at the time of
immunisation are avoided, and the site of the injection is not harmfully
effected. A solution containing an antigen and hyaluronic acid does not present
the advantageous properties of the particulate complex according to the invention.

Examples of allergens, which can form a particulate complex with
hyaluronic acid in accordance with the invention, are serum albumins.
foodstuff allergens, mould extracts, animal epitel extracts, caracein extracts, various pollen extracts, etc. Examples of vaccines, which can be complex bonded to hyaluronic acid in a corresponding manner, are i.a. pertussis, tetanus, etc.

According to the invention the complex antigen/hyaluronic acid has the form of a finely divided powder, which is suited for injection in the form of a suspension in any suitable physiologically acceptable liquid medium. The particulate complex preferably has a particle size of from 0.1 to 200 μ, especially from 1 to 100 μ, and in particular from 5 to 60 μ. The complex can be regarded as a difficultly soluble salt between the antigenic protein and the hyaluronic acid.

According to another aspect of the invention the particulate complex antigen/hyaluronic acid is prepared by reacting hyaluronic acid with the respective antigen in an aqueous reaction medium, lowering the pH of the reaction mixture until the desired antigen/hyaluronic acid complex falls out as a finely divided precipitate, and recovering the precipitated complex. When necessary, the precipitate is washed so that the desired complex will be essentially free from free antigen. In order to facilitate the precipitation a physiologically acceptable, non-antigenic protein such as, for example, albumin from the animal (including man) to be immunised can be added.

According to a preferred embodiment of the method according to the invention the precipitate obtained is treated with an aqueous solvent, for example a lower alkanol such as methanol or ethanol. It has surprisingly been found that such treatment even more improves the properties of the complex especially by improving the depot effect. After this treatment the precipitate is worked up and dried in any suitable manner.

The hyaluronic acid used as the starting material preferably has molecular weight of at least 750,000, and it is preferred to use a salt thereof for example the sodium salt. The relative amounts of the components of th complex are preferably adjusted such that the dry particulate comple contains hyaluronic acid and antigen in a weight ratio of from 1:2 to 1:20, e.; from 1:2 to 1:12, in particular from 1:3 to 1:9. If a non-antigenic proteins used for facilitating the precipitation of the complex, the same may conta up to about 50 percent by weight of this protein. An especially suitable type hyaluronic acid is the one described in our U.S. patent No. 4,151,973 al having the following characteristics: a mean molecular weight of at lea about 750,000, a protein content lower than 0.5 percent by weight, a U absorbance for a 1% solution of the sodium salt lower than 3.0 at 257 nm a lower than 2.0 at 280 nm, and a kinetic viscosity greater than 1000 centisto for a 1% solution of sodium salt in physiological buffer.

As mentioned above injection of the particulate complex according to the invention results in a very strong and long lasting effect already for very low doses of the antigen, these doses being lower than when using only the antigen in question. The dosage, which varies from antigen to antigen, is chosen with regard to the needs and desires in the individual case. Generally microgram amounts are sufficient, for example 0.5 µg to 1,000 µg of the complex per injection (corresponding to, for example, 0.3 to 600 µg of antigenic protein and 0.2 to 250 µg of hyaluronic acid per injection).

According to another aspect of the invention the novel particulate complex of an antigen and hyaluronic acid is used for preparing antisera containing a high titer of antibodies against the respective antigen. Such antisera can be used for e.g. immunological determinations. The invention thus also relates to a method for producing high titer antisera by injecting the particulate complex according to the invention into a host animal, taking blood from the host animal when essential amounts of antibodies have been formed, and preparing the serum in conventional manner.

The invention will be illustrated further in the following Examples which, however, in no way are intended to restrict the scope of the invention. The solution of hyaluronic acid used in the Examples had the following properties: molecular weight $3.7 \times 10^6$, limiting viscosity 2.380 to 2.470 ml/g, protein content 0.02%.

## Example I

10 ml of a solution of hyaluronic acid in 0.1M NaCl (2.5 mg/ml) were diluted to 100 ml with 0.1M NaCl. 100 ml of dog albumin solution (1 mg/ml) were added while stirring. Diluted acetic acid solution, prepared by diluting 0.5 ml of a 5% acetic acid solution to 20 ml with distilled water, was added dropwise while agitating with a magnetic stirrer until pH 4.2, the complex precipitating in the form of a fine precipitate.

The precipitated complex was centrifuged and washed once with water. After dissolution in 0.1% $Na_2CO_3$ (20 ml) the complex was reprecipitated by acidifying to pH 4.45 with diluted acetic acid solution as above. The suspension obtained was sprayed into ethanol (99.5%) while stirring. The precipitate was left in ethanol for 2 to 24 hours, depending on the desired degree of dissolution. Finally, the product was filtered off, dried in vacuum at room temperature, and freeze dried as single-unit packs.

Yield: 30 to 100 mg of a dry, finely divided powder which is difficultly soluble in 0.9% sodium chloride solution and which contains hyaluronic acid and dog albumin in a weight ratio of 1:6.

## Example 2

The procedure of Example 1 was repeated using an egg albumin solution instead of the dog albumin solution. The egg albumin/hyaluronic acid complex obtained consisted of a dry, finely divided powder, which was difficultly soluble in 0.9% sodium chloride solution and contained hyaluronic acid and egg albumin in a weight ratio of 1:4.6.

## Example 3

A preparation for immunisation suitable for e.g. subcutaneous injection was prepared as follows:

150 ml of a sterile filtered birch pollen extract (Spectralgen®, available from Pharmacia AB, Uppsala, Sweden) containing 1.8 mg extract/ml were mixed with 150 ml of hyaluronic solution (0.25 mg/ml) and 75 ml distilled water. 0.04M acetic acid solution was added under agitation until the pH was 3.70 and the hyaluronic acid/birch pollen extract precipitated as a fine precipitate. Ethanol (99.5%) was then added under agitation until the ethanol concentration was 30%. Agitation was continued for 4 h at 67°C, and the suspension was then filtrated. The filtered precipitate was washed several times with distilled water, then suspended in a 3% mannitol solution and freeze-dried into single-dose packs.

Yield: 80-100 mg of a particulate complex (45-70% based on protein, 60-70% based on hyaluronic acid), which is difficultly soluble in 0.9% saline solution and contains hyaluronic acid and birch pollen extract in a weight ratio of 1:2.6.

## Example 4

A suspension suitable for injection, especially subcutaneous injection, of the particulate hyaluronic acid/dog albumin complex prepared according to Example 1, was prepared as follows:

13.6 g of the dried complex according to Example 1 was suspended in 1 ml of 0.9% NaCl plus 0.05% Tween®20 (Solution A). The suspension was left for one hour at 23°C and then for 20 hours for 4°C. The suspension was centrifuged and the supernatant was discarded. The recovered precipitate was washed with 200 µl of Solution A and resuspended in 3 ml of the same solution.

Suspensions for injection of the particulate egg albumin/hyaluronic acid complex prepared according to Example 2 and of the particulate birch pollen/hyaluronic acid complex prepared according to Example 3 were prepared in the same manner.

## Example 5

A suspension for injection, prepared in accordance with Example 4, of the particulate dog serum albumin/hyaluronic acid complex (HA-DSA) was in immunisation tests on rabbit compared with a different preparation of dog serum albumin (DSA), namely DSA dissolved in sodium chloride solution (DSA-salt). Both preparations were administered with the same amount of DSA, namely 100 μg/0.5 ml.

In the test the respective preparation was injected once subcutaneously under the neck skin of white New Zealand rabbits, with five animals per preparation. Serum samples were taken before the immunisation and 5, 15, 25 and 40 days after the same. The samples were examined with regard to anti-DSA antibodies by passive hemagglutination. Two types of red cells were sensitized with DSA by glutaric aldehyde coupling, viz. Guinea pig cells (GPC) and human group 0 cells (HRC).

The test results are given in Table I below. As can be seen the DSA-sodium chloride solution did not induce any formation of antibodies. In contrast thereto the DSA complex according to the invention produced a strong response with high titers, which furthermore remained over the entire test period.

### Table I

Immunogenicity of two different preparations of dog serum albumin (DSA) in rabbits. Anti-DSA antibodies were measured by passive hemagglutination before and up to 40 days after the immunisation.

| Type of DSA pre-paration | Animal No. | Titers of anti-DSA antibodies | | | | | | | | | |
|---|---|---|---|---|---|---|---|---|---|---|---|
| | | Before | | 5 days | | 15 days | | 25 days | | 40 days | |
| | | GPC | HRC | GPC | HRC | GPC | HRC | GPC | HRC | GPC | HRC |
| HA-DSA [1)] | K-1 | 0 | 2 | 0 | 0 | 256 | 256 | 64 | 64 | 32 | 32 |
| | K-2 | 1 | 0 | 1 | 0 | 32 | 32 | 16 | 16 | 8 | 2 |
| | K-5 | 2 | 0 | 128 | 4 | 2 | 2 | 2 | 1 | 2 | 1 |
| | K-6 | 0 | 0 | 0 | 0 | 32 | 32 | 32 | 16 | 16 | 3 |
| | K-7 | 0 | 0 | 2 | 1 | 64 | 64 | – | – | 16 | 15 |
| DSA-saline | K-55 | 0 | 0 | 0 | 0 | 1 | 0 | 0 | 0 | 0 | 5 |
| | K-85 | 0 | 0 | 0 | 0 | 0 | 0 | 0 | 0 | 0 | 5 |
| | K-0661 | 0 | 0 | 0 | 0 | 0 | 0 | 0 | 0 | 0 | 0 |
| | K-855 | 0 | 0 | 0 | 0 | 0 | 0 | 0 | 0 | 0 | 9 |
| | K-866 | 1 | 0 | 0 | 0 | 0 | 0 | 0 | 0 | 0 | 2 |

1)     DSA/hyaluronic acid complex according to the invention.

The titers are given as reciprocals of the highest serum dilution giving hemagglutination.

All sera were tested on DSA-sensitized red cells from Guinea pigs (GPC) and humans (HRC).

## Example 6

The test procedure of Example 5 was repeated on rats using three different DSA-preparations, viz. 1) DSA in phys. saline, 2) phys. saline solution of DSA and HA, and 3) a suspension of the particulate DSA–HA complex of the present invention. In all three cases the rats were immunised on days 3 and 13, each time with a dose of 10 µg DSA. Anti–DSA antibody levels were measured on days 0, 13 and 27. The test results, which were expressed as log 2 titer (mean of 6 rats), are reported in Table II below.  It can be seen that the present particulate DSA–HA complex resulted in considerably increased and extended antibody titers, not only compared to the DSA–saline solution, but also compared to the solution containing both DSA and HA.

## Table II

Immunicity of DSA-preparations in rat. Passive hemagglutination values expressed as log 2 titers (mean of 6 animals).

| Type of DSA preparation | Anti-DSA antibodies | | |
|---|---|---|---|
| | Day 0 | Day 13 | Day 27 |
| DSA-saline | 5 | 4.17 | 5.83 |
| DSA-HA solution | 5 | 5.17 | 8.6 |
| Suspension of particulate complex of DSA-HA | 5 | 5.33 | 14.67 |

## Example 7

In order to simulate "therapeutic conditions" in humans tests were made with ovalbumin (OA) primed rats as follows:

Pigmented Lister rats were used with groups of 9 animals per test group. The rats were pretreated with a single dose of 1 µg OA in Freunds complete adjuvant. Three weeks later a single dose of the tested preparations was given. Serum samples were taken before the pre-treatment, immediately before the treatment with the test preparationss and one week thereafter. The response on the treatment was measured by homologeous passive hemagglutination and with RAST on rat. (Radio Allergo Sorbent Test – see e.g. Bennich et al, Evaluation of basic serum JgE levels and the JgE antibody response in the rat by radio immuno assays, Imm. Rev. Vol. 41, p. 261-287, 1978.)

The results of the tests are given in Tables III and IV below, wherein Table III indicates the titers of hemagglutinating anti-ovalbumin antibodies (IgG + IgA + IgM), whereas the anti-ovalbumin antibody titers of the IgE class are indicated in Table IV.

<u>Table III</u>

| Type of OA | number | Anti-OA-titer – mean value | | |
|---|---|---|---|---|
| | | before priming | immediately before treatment | 1 weak after treatment |
| OA-HA- omplex (100 μg) | 8 | 6 | 33 | 336 |
| phys. saline | 9 | 4 | 21 | 11 |

<u>Table IV</u>

Titer of circulating anti-ovalbumin antibodies of the IgE type. Adjuvant effect for particulate ovalbumin/hyaluronic acid complex (Treat) in rats primed (Imm.) with ovalbumin.

| | Titer expressed as pulses /min. [x)] | | | | |
|---|---|---|---|---|---|
| | week 0 | | week 3 | | week 4 |
| OA-HA-complex (100 μg) | 6438 | Imm. | 12763 | Treatm. | 12427 |
| OA-HA-complex (14 μg) | 4287 | | 12637 | | 14810 |
| phys. saline | 4936 | | 9110 | | 5873 |

x) Mean of 9 rats.

1

## CLAIMS

1.      An immunisation agent containing proteinous antigen, wherein the antigen is present in the form of a particulate complex with hyaluronic acid.

2.      An agent according to claim 1, wherein the complex is a co precipitate of the antigen and hyaluronic acid.

3.      An agent according to claim 1 or 2, wherein the particulate comple has a particle size of from about 0.1 μ to 200 μ.

4.      An agent according to claim 1, 2 or 3, wherein the particulat complex is suspended in a physiologically acceptable, especially aqueou medium.

5.      An agent according to claim 1, 2, 3 or 4, further comprising physiologically acceptable, non-antigenic protein.

6.      An agent according to any one of claims 1 to 5, wherein th particulate complex contains hyaluronic acid and antigen in a weight ratio c from about 1:2 to about 1:20.

7.      An agent according to any one of claims 1 to 6, wherein the antigen an allergen or a vaccine.

8.      A method of preparing an immunization agent containing proteinou antigen in the form of a particulate complex with hyaluronic acid, comprisir the steps of reacting hyaluronic acid with the respective antigenic protein the presence of an aqueous reaction medium, lowering the pH value of tł reaction mixture until a finely divided precipitate of the desired complex formed, and recovering the precipitate from the reaction mixture.

9.      A method according to claim 8, wherein the precipitate formed treated with an inert organic solvent that can be mixed with water.

10.      A method of producing antisera of a high titer, comprising the ste of injecting an agent according to claim 1 into a host animal, taking bloc from the host animal when the desired amount of antibodies has been forme and working up the serum in conventional manner.